# EUROPEAN PATENT APPLICATION

(11) **EP 0 992 582 A2**
(43) Date of publication of application: **12.04.2000**
(21) Application number: 99307832.8
(22) Date of filing: 05.10.1999
(51) Int. Cl.: C12N 15/09, A61K 39/00, C12N 15/62

(54) **Schistosoma recombinant fusion protein vaccine**

(30) Priority: 07.10.1998 GB 9821821
(71) Applicant: University of Wales, Bangor, Bangor, Gwynedd LL57 2DG (GB)
(72) Inventor: Doenhoff, Michael, Bangor, Gwyned LL57 2DG (GB); Sayers, Jon, Univ. Div. of Molecular and Gen. Med., Sheffield S10 2JF (GB)
(74) Representative: Brandon, Paul Laurence

(57) **Abstract**

A vaccine for eliciting immunity against Schistosoma parasites, comprises a recombinant fusion protein capable of comprising the 27/28 kDa cercarial elastase sequence of S. mansoni or an active fragment, homologue or variant thereof, fused to a suitable bacterial, phage or viral protein, together with a pharmaceutically acceptable excipient or carrier. The vaccine can be used to combat S. mansoni, S. japonicum and/or S. haematobium in mammals, especially humans.

## Description

This invention relates to a recombinant fusion protein capable of eliciting immunity against Schistosoma parasites and vaccines incorporating such proteins.

Several hundred million people in the tropics have schistosomiasis or are at risk from infection, and the problem is on the increase due to development of water resources for agriculture and industry. The disease is caused by species of trematode worms, the adults of which live for many years in blood vessels of the portal system or urinary tract. A large proportion of the eggs produced by the worms remain in the body and induce inflammatory reactions which block blood and/or urine flow.

The available methods of controlling schistosomes include treating infested water to kill snail intermediate hosts, or treatment of infected patients with drugs. Chemoprophylaxis is impractical and no commercial vaccine exists. Development of an effective vaccine for this disease is considered a priority by the World Health Organization.

Infection of human hosts begins when larvae (cercariae) produced by infected snails come into contact with human skin. Penetration of skin by the larvae is highly dependent on a potent serine protease (the 'cercarial elastase') which the larvae excrete to digest skin constituents while migrating towards blood capillaries that lie beneath the basement membrane.

There are different recognized species of schistosomes. Three of them are important parasites of man S. mansoni, S. haematobium and S. japonicum. The species are usually considered to belong to three groups: those with a lateral egg spine as typified by S. mansoni; those with a terminal egg spine as S. haematobium and those with round minutely spined eggs as S. japonicum.

**S. mansoni**, the cause of intestinal schistosomiasis in man, occurs in Africa and Central and South America. The eggs are excreted with the faeces. The snail intermediate hosts belong to the genus Biomphalaria. Humans seem to be the major reservoir of the disease, but there are reports of infection with S. mansoni in a wide range of mammalian species. These include baboons and rats and these could play important roles in the epidemiology of S. mansoni by acting as reservoir hosts.

**S. japonicum** is responsible for a chronic form of intestinal schistosomiasis which affects both man and domestic animals and is found in the Far East. Animals appear to play a very important role in the epidemiology of the disease. The intermediate hosts belong to the genus Oncomelania.

**S. haematobium** is almost exclusively a parasite of man. The parasite inhabits the plexus of the veins around the bladder and eggs with terminal spine are eliminated mostly with urine although occasionally they are found in the faeces. The distribution of S. haematobium is restricted to Africa and the Middle East. Different species of snail of the genus Bulinus are involved in the transmission cycle.

Workers investigating the cercarial elastase have suggested its use as a serological reagent for determining levels of cercarial exposure (Ramzy et al, Am. J. Trop. Med. Hyg., 56, (1997), 668-673). However, the S. mansoni cercarial elastase in its native form has been found to be a very poor immunogen in so far as only a small minority of mice produced specific antibody against it, even after multiple immunizations (Darani et al, Parasitology, 115, (1997), 237-247).

We have now discovered that, in contrast to the poor immunogenic performance of the native form of S. mansoni cercarial elastase, fusion proteins containing the cDNA gene expression product and certain fragments thereof induce marked antibody response against infections with Schistosoma parasites.

According to this invention we provide a vaccine comprising a recombinant fusion protein capable of eliciting immunity against Schistosoma parasites, comprising the 27/28 kDa cercarial elastase sequence of S. mansoni or an active fragment, homologue or variant thereof, fused to a suitable bacterial, phage or viral protein, together with a pharmaceutically acceptable excipient or carrier.

Suitably the fusion protein comprises the amino acids coding for the nucleotide of the cercarial elastase gene of S. mansoni (Seq. I.D. 1) or a homologue or variant thereof. This sequence was determined by Newport et al (J. Biol Chem, 263, (1988), 13179-13184).

Preferably the fusion protein comprises at least the amino acid sequence coding for exon 2 of the S. mansoni cercarial elastase gene as herein defined (Seq. I.D. 2). A preferred fusion protein comprises at least amino acid residues 136 to 151 of the S. mansoni cercarial elastase molecule. Alternatively, this preferred fusion protein can be defined as comprising a sequence of at least 16 amino acids including the sequence: V G Y G R D D N D R D P S R K N (Seq. I.D. 3) fused to a suitable bacterial, phage or viral protein.

The fused protein is suitably a glutathione-S-transferase, for example the 28 kDa glutathione-S-transferase of S. japonicum. However, other commercially available bacterial, phage or viral proteins suitable for use in recombinant DNA methods can be employed. Examples are cholera toxin and keyhole limpet haemocyanin.

The terms 'variant', 'homologue' or 'fragment' include any substitution of, variation of, modification of, replacement of, deletion of, or addition of one or more amino acid(s) from or to the sequence, providing the resultant fusion protein retains its ability to elicit immunity.

The term "pharmaceutically acceptable excipient or carrier" includes any suitable medium for administration, such as oral administration or administration via injection. Such mediums are well known in the art, such as water or isotonic saline, with or without the addition of suitable adjuvants.

Further aspects and advantages of the invention will be apparent from the following detailed description with nonlimiting Examples.

### Brief Description of the Figures

Figure 1 gives the consensus sequence of the Schistosoma mansoni cercarial elastase gene (Seq. I.D. 1).

Figure 2 is a diagrammatic representation of the production of Construct pGEXCEL.

Figure 3 is an SDS-PAGE illustrating purification of recombinant protein Sm30-GST.

Figure 4 shows Western blot reactivity of a serum from rabbit immunized with Sm30-GST.

Figures 5 and 6 show Western blots from mice immunized with Sm30-GST.

Figure 7 is a Western blot showing cross-reactivity between cercarial elastases of different schistosome species.

Figure 8 is a Western blot showing reactivity from mice immunized with Sm30-GST and, respectively, with exons 1,2 and 3 of Sm30 fused to GST.

### Materials and Methods Employed in the Examples

### Snail maintenance

For the maintenance of all S. mansoni strains Biomphalaria Spp. snails were used. These intermediate hosts of S. mansoni were kept in water-filled plastic tanks (15 litres capacity) in a room which was maintained at 26-28°C and illuminated on a 12 hour diurnal cycle with warm white fluorescent tubes. The water was supplied through non-copper piping and dechlorinated by leaving it to stand for 24 hours, which also allowed for temperature equilibration. The snails were fed daily on commercial pelleted rabbit diet and the water supplemented with Nolan-Carriker salt solution at a concentration of lml per litre. The snail tanks were kept fully aerated using airstones connected by plastic aquarium tubing to a constant air supply.

Adult breeding snails were kept in tanks at an approximate density of one snail per litre of water, the tanks being disturbed as little as possible. The water was changed when it became cloudy or when unwanted microfauna and flora developed. All cultures contained a small number of water fleas, which were believed to provide a better environment for the snails by keeping the water clean.

### Parasite production (for infecting mice)

### Parasite strains

Different strains of the parasite S. mansoni such as geographic isolates from Puerto Rico (PR), Brazil, Egypt and Kenya were maintained in the laboratory. For the production of parasitic material the cercariae from these isolates were pooled in varying proportions. S. mansoni PR, which was isolated in 1964 from the eggs excreted by a human patient in Puerto Rico, was used for experimental procedures requiring quantitive parasite recovery.

### Larval production

### (i) Snail infection

For the infection of snails, S. mansoni eggs obtained from the livers of heavily infected mice were killed 45 days or more after infection. The livers were macerated through a 500µm sieve into a urine flask. The sieve was repeatedly flushed with isotonic saline and the sieved material allowed to settle at 4°C. The supernatant was discarded and the sediment repeatedly washed with saline until non-sedimenting material had been removed. The sediment was then added to 10ml distilled water and eggs allowed to hatch by placing them in a Petri dish under a desk lamp. The hatched miracidia were added to a tank containing Biomphalaria glabrata snails at 26°C. The tank was left undisturbed for a minimum of 5 hours.

Infected snails became patent approximately 30 days after exposure to miracidia. They were placed in dark for two days, after which time the snails were screened for infection. Individual snails were placed in small glass tubes containing approximately 5ml of aquarium water. These were then illuminated and warmed to 30°C in a water bath for 3 hours. The water was examined for the presence of cercariae. Individual snails shedding cercariae were pooled and kept in the dark until cercariae were required. Snails which were not deemed to be infected were re-scanned a week later and discarded if they were not shedding at this stage.

### (ii) Harvesting and concentration of cercariae

When cercariae were required, infected snails were removed from the tanks which had been kept in darkness. The snails were placed in beakers containing copper free water and allowed to shed their cercariae under artificial light in a water bath maintained at 30°C. Snails were left to shed for an hour after which the cercariae were harvested and fresh water placed on the snails. The first and second sheds were pooled and the snails placed back in tanks in the dark. Snails were generally shed twice a week under such conditions.

Cercariae were either used at the densities at which they were harvested or concentrated before use. Cercarial densities were measured by taking a number of appropriately sized aliquots and counting the number of intact cercariae present in each aliquot under a dissecting microscope after staining with Lugol's iodine. In cases where the cercariae had to be concentrated this was achieved on a Millipore concentration apparatus (Millipore Corp., Massachusetts, USA) using a vacuum pump to gently remove excess water through a 8µm pore size, 47cm Millipore filter (Anderman and Co. Ltd). The positively phototropic cercariae were attracted away from the filter by overhead illumination. S. mansoni cercariae were used for infection of experimental animals within 3 hours of their emergence from the snails.

### Concentration of cercariae and preparation of cercarial homogenates

S mansoni cercariae (as used in Example 6) and S. haematobium cercariae were concentrated by filtration and gravity sedimentation at 4°C. They were resuspended in water at a concentration of 5 x 10⁵/ml and disrupted by sonication until no intact cercarial bodies remained (see Doenhoff et al, Trans Roy Soc Trop Med Hyg, 75 41-54, 1981).

### Cercarial Transformation Fluid

The preparation of CTF required for immunization was obtained as described in Example 6. The suspension of live cercariae which had been concentrated to a volume of 10ml as described above was placed in universal tubes and left to stand for 1 hour at 4°C. The supernatant was discarded and 2ml of medium 199 was added to the pellet of packed cercariae of several tubes. The suspension was passed ten times backwards and forwards through a syringe with a 21g needle. The suspension was incubated at 3°C for 2-3 hours, after which it was centrifuged at 2000g and the supernatant aliquoted and stored at -20°C until required as unconcentrated CTF. Before use it was concentrated several fold using ultra filtration equipment, after which the total protein of the concentrated CTF was about lmg/ml.

### Challenge of Mice with S. mansoni.

### a) Percutaneous infection of mice

The technique employed was that of Doenhoff et al (J. J. Helminthology, 52 (1978), 173-186). The S. mansoni cercariae were obtained as described above under Methods and Materials. Mice were anaesthetized with sodium pentobarbitone (0.06mg/g body weight Sagatal, May and Baker Ltd, Dagenham, England) administrated by intra-peritoneal injection. Abdominal skin was shaved and the mice placed on their backs in wooden holding racks and gently secured with "Sellotape". The shaved skin was moistened with aquarium water and a nickel plated or plastic ring with an internal capacity to hold approximately lml, was placed on it. The ring was secured with "Sellotape". Volumes of cercarial suspension adjusted to give the required numbers of cercariae per mouse were placed in the rings. If the volume was less than 0.5ml, aquarium water was added to bring the total to this volume. The cercariae were then left to penetrate for 30 minutes. Penetration rates of over 90% were obtained with these parasites using this method, as judged by the numbers of residual cercariae which were found in the water after infection. Following cercarial penetration the mice were removed from the apparatus and kept in a warming box until they recovered.

### b) Portal perfusion of schistosome-infected animals

Recovery of adult schistosome worms was achieved by the portal vein perfusion technique of Smithers and Terry Parasitology, 65, 695 (1965) as adapted by Doenhoff et al, J. Helminthology, 52, 173 (1978). Infected mice were sacrificed under 30mg/mouse pentobarbitone-induced anaesthesia (Sagatal) containing 25 units heparin/ml. The thoracic and abdominal cavities were opened. Mice were then suspended on a vertical perspex board by means of clips to their legs. The liver lobes were suspended over a funnel leading to a 20ml "universal tube" (Sterilin). The intestines were lifted to reveal the hepatic portal vein which was cut at the point where it enters the liver. 30ml of perfusion fluid (8.6g NaCl, 15g trisodium citrate, 200 units Heparin and 0.2g Merthiolate per litre of distilled water) were injected into the right ventricle of the heart using a 50ml syringe (Sterilin) with a 15 gauge needle (Yale Microlance, Becton, Dickinson and Co., Ireland). The livers and intestines were gently massaged to facilitate worm expulsion. Perfusion was complete when the mesenteric veins and liver were clear of blood. On completion the organs of the mouse and funnel were checked for any adhering worms. Worms were allowed to settle in the universal containers and the excess perfusion fluid was removed and replaced until the suspensions were relatively free of red blood cells. The worms at this stage were placed into gridded plastic Petri dishes for counting under the dissecting microscope.

### Tissue Egg Counts

The method used was that described by Cheever, Bull. WHO, 39, 328-331 (1968) adapted as in Doenhoff et al., J. Helminthology, 52, 173-186 (1978). The liver tissue egg count in all cases is given as the number of the eggs in the total liver. Livers for tissue egg count estimation were digested at 37°C in 20ml of 5% potassium hydroxide for 18 hours. After this the digest fluid was poured into a 100ml beaker and vigorously stirred. Three 50ul aliquots were taken using an automatic pipette and each aliquot was placed on separate microscope slide under a cover slip. The number of eggs in each aliquot was counted under x 10 magnification. The mean value for the three samples was calculated, and the total number of eggs in the liver tissue was calculated from the mean. If the counts of any individual aliquot deviated more than 10% from the mean of the other two, a further aliquot was counted.

### SDS-Polyacrylamide Gel Electrophoresis (SDS-PAGE) (Method A)

SDS-PAGE was performed using a discontinuous buffer system and slab gel apparatus. A 10% (w/v) acrylamide SDS resolving gel was prepared using 3.33ml of 30% (w/v) acrylamide, 0.8% bisacrylamide [37.5:1] solution (NBL Gene Sciences Ltd), 2ml of 500mM Tris-bicine [pH 8.3], 100µl of 10% (w/v) SDS solution and 4.35ml of distilled water. Immediately before pouring the gel, 200µl of ammonium peroxydisulphate (25mg/ml) and 16µl of TEMED were added to the mixture. The gel was poured using a 1-5ml adjustable pipette into the slab gel apparatus, which consisted of two glass plates (one plate measuring 12.6cm x 12.5cm x 4mm, and the other plate measuring 12.6cm x 11.4cm x 4mm) and three spacers (measuring 12.6cm x 1cm x 1mm) secured together with petroleum jelly and three board clips. A lml volume of butanol was added to the surface of the gel which was left to polymerise for 30 minutes.

A 5% (w/v) acrylamide stacking gel was prepared using 500µl of 30% (w/v) acrylamide, 0.8% bisacrylamide [37.5:1] solution (NBL Gene Sciences Ltd), 1.5ml of 250mM Tris-HCl [pH 6.8], 30µl of 10% (w/v) SDS solution and 1 ml of distilled water. The butanol was removed from the polymerised resolving gel, and immediately before pouring the stacking gel, 15µl of ammonium peroxydisulphate (25mg/ml) and 5µl TEMED were added to the mixture. The stacking gel was poured onto the surface of the resolving gel using an adjustable pipette. A nine-toothed comb (each tooth measuring 6mm x 9mm x lmm) was placed in the top of the gel which was then left to polymerise for 30 minutes. The prepared gel and glass plates were placed in an upright position into a perspex SDS-PAGE tank containing two running buffer reservoirs. A 250ml volume of running buffer (100mM Tris, 100mM bicine, 0.1% (w/v) SDS) was added to the tank and air bubbles were removed. Protein samples were prepared for electrophoresis using approximately 2-5µg of protein per lane. To lyse bacterial samples, cell pellets from 200µl of culture were resuspended in 50µl of cell lysis buffer (2% (w/v) SDS, 10mM EDTA [pH 8.0], 50µg/ml PMSF, freshly prepared) and boiled for 3 minutes. A 15µl aliquot of cell lysate or prepared protein was added to 15µl of loading buffer (100mM Tris.HCl [pH6.8], 200mM DTT, 20% (v/v) glycerol, 10mM EDTA [pH 7.0] 4% (w/v) SDS, 0.2% (w/v) bromophenol blue). A molecular weight marker sample was also prepared, using 5µl of a Promega low or medium weight protein marker solution and 15µl of loading buffer (as above). The samples were boiled for a further 3 minutes before loading onto the electrophoresis gel.

A current of 25mA was applied for approximately 3-4 hours, until the bromophenol blue dye was about 1cm from the bottom of the resolving gel. The acrylamide gel was removed from the electrophoresis apparatus and incubated in Coomassie blue staining solution (0.4% (w/v) Coomassie brilliant blue R, 0.1% (w/v) Coomassie brilliant blue G, 42% (v/v) ethanol, 5% (v/v) methanol, 10% (v/v) glacial acetic acid) for 30 minutes at 50°C. The gel was washed in distilled water for 3 minutes, then incubated in destaining solution (45% (v/v) methanol, 10% (v/v) glacial acetic acid) for 20 hours at room temperature, changing the buffer three times.

### SDS-PAGE (METHOD B)

### Sodium dodecyl sulphate polyacrylamide gel electrophoresis (SDS-PAGE)

Paired glass plates (8.2 x 10.2 x 0.1cm and 7.2 x 10.2 x 0.1cm) separated by 0.75mm teflon spacers were clamped in a cassette and mounted on a holder to provide a chamber to contain the acrylamide. Resolving gel was made up to required acrylamide concentration:

| | | |
|---|---|---|
| Percentage acrylamide | 12% | 15% |
| Resolving buffer (ml) | 2.5 | 2.5 |
| Acrylamide (ml) | 3.8 | 4.8 |
| Distilled water (ml) | 3.7 | 2.7 |

After degassing and warming at 37°C for ten minutes, the setting process was initiated by adding 100µl ammonium persulfate (50mg/ml), 100µl SDS (10%) and 10µl Temed. 3.5ml of this solution were pipetted into the chamber and a small quantity of N-butanol layered above the setting gel. Once set the butanol was removed and the top surface of the gel was rinsed with distilled water.

Acrylamide concentration in the stacking gel was 3%. It was made with 2.5ml stacking buffer, 0.5ml acrylamide and 2ml distilled water. The mixture was degassed and warmed, then 75µl of ammonium persulfate, 50µl SDS, 5µl of phenol red and 10µl of Temed were added. The gel was poured on the top of the resolving gel and combs were inserted. The gel was left to set after which time the comb was carefully removed and the wells rinsed with distilled water.

Pairs of cassettes were attached to the electrode insert of the electrophoresis apparatus, thus forming an upper reservoir containing the cathode, the whole of which was in turn suspended in a tank to form a lower reservoir containing the anode. Running buffer was poured in the upper reservoir and lower buffer in the lower reservoir.

Antigen samples were prepared by adding ¼ volume of loading buffer and boiling for 2 minutes. A small amount of bromophenol blue was added to visualise the samples. These were loaded in the individual lanes (10-15µl/well) or troughs (150µl/continuous well).

Molecular weight standards were applied to the end lane for reference. These contained: 14kD (cytochrome C), 20kD (soybean trypsin inhibitor), 30kD (carbonic anhydrase), 43kD (ovalbumin), 67kD (bovine serum albumin) and 94kD (phosphorylase).

The apparatus was connected to a power pack which was set to deliver 100 volts/gel. Electrophoresis was run until the bromophenol blue in the sample buffer had reached the lower edge of the gel, usually after approximately 40 minutes.

### Coomassie Blue-staining of gels

Gels were removed from the cassettes and immersed in 0.1% solution of Coomassie Blue R250 in distilled water: methanol: glacial acetic acid (5:5:2 by volume). The gel was incubated overnight and then placed in a destaining solution; distilled water: ethanol: glacial acetic acid (63:30:7 by volume). The destain was removed and replaced until background colour in the gel had been removed.

### Western Blotting (Method A)

SDS-PAGE was performed using selected samples of cell lysates, schistosome antigens and horse albumin (Sigma). Proteins were electrophoretically transferred onto Hybond-C Super nitro-cellulose paper (Amersham-USB, UK) (Burnette, 1981) using a BioRad (Hemel Hempstead, Herts, UK) transblot electroblotter, filled with blotting buffer (25mM Tris, 190mM glycine, 20% (v/v) methanol) . A current of 40mA was applied for 2 hours, regulating the blotting buffer temperature with an LKB 2209 Multitemp cooling unit set at 4°C.

When the transfer had been completed, the protein molecular weight marker lanes were removed and stained as described below. The remaining blots were incubated in 100ml of TTBS (20mM Tris-HCl [pH 8.0], 300mM NaCl, 0.1% (v/v) Tween 20) with lg dried milk powder for 1 hour, then washed with TTBS. This was followed by incubation in 1:400 dilution of rabbit serum or 1:200 dilution of mouse serum, in TTBS for 3 hours. After washing with TTBS, the blots were incubated for 2 hours in 1:1000 dilution of peroxidase-labelled goat anti-rabbit or goat anti-mouse IgG conjugate serum (Nordic Immunoconjugates, Maidenhead, Berkshire, UK) in TTBS. After washing in TTBS, the blots were placed in developing solution (20mg 4-chloro-1-naphthol in 4ml of methanol, 20ml of TBS (20mM Tris-HCI [pH 8.0], 300mM NaCl) and 10µl of 30% (v/v) hydrogen peroxide) for 5 minutes, then washed in distilled water.

### Staining Molecular Weight Markers from Western Blots

Following electrotransfer to nitro-cellulose paper, the protein molecular weight marker lanes were removed from prepared blots and were subjected to three 5 minute washes in PBS solution (137mM NaCl, 2.68mM KCl, 4.3mM NaH₂PO₄, 1.47mM KH₂PO₄, pH 7.4). This was followed by a 30 minute wash in PBS-Tween solution (PBS as above plus 0.3% (v/v) Tween 20), three 5 minute washes in PBS (as above) and two 5 minute washes in distilled water. The nitro-cellulose strips were incubated in Protogold protein staining solution (British Biocell International, UK) for 3 hours, then in distilled water for 3 hours. The strips were allowed to dry.

### Western blotting (Method B)

### A. Electrotransfer of proteins onto nitrocellulose paper (NCP)

A sheet of NCP was cut and soaked in electroblotting buffer. Four pieces of Whatman filter paper were treated in the same way. A blot sandwich was assembled in the following order: Perforated perspex - Scotch Brite pad - 2 Whatman filter papers - SDS-PAGE gel - NCP paper - 2 Whatman filter papers - Scotch Brite pad.

An electroblot tank was filled with transfer buffer, the assembled sandwich was clamped shut and placed inside such that the gel was cathodal to the NCP paper. A current of 0.44 amps was applied to the tank for 3 hours. Following electrotransfer, the sandwich was removed from the tank and the strips of NCP were either stained with Protogold or treated for immunoblotting.

### B. Protein staining in NCP papers

Colloidal gold sol was used to stain molecular weight markers on NCP. This procedure was followed:
3 x 5 minute washes in PBS
20 minutes incubation at 37°C in PBS and Tween 20
3 x 5 minute washes in PBS and Tween 20
1 wash in distilled water
Staining overnight in Protogold

### C. Immunoblotting

NCP paper was placed in a solution of 1% milk powder in TTBS (transblotting solution containing 0.1% Tween 20) for one hour at room temperature under agitation. This procedure blocks free protein binding sites on the NCP with an irrelevant known protein. Afterwards, the NCP was washed 3 times for five minutes in TTBS and then each NCP paper strip was incubated for four hours with appropriate dilution of the antiserum with agitation. The strips were washed with 3 washes of TTBS and incubated for two hours with 1/1000 dilution of an enzyme- conjugated anti-species IgG antibody. Finally the NCP strips were washed with three changes of TTBS over 15 minutes and incubated in enzyme substrate solution (20ml TBS, 20mg 4-chloro-1-naphthol in 4ml methanol and 10µl hydrogen peroxide).

### Enzyme linked immunosorbent assay (ELISA)

Dynatech M29a flat-bottomed polystyrene microtitre plates were coated with antigen diluted in coating buffer (pH 9.6) at 37°C for one hour and at 4°C overnight. Into each well 100µl of reagent was pipetted.

The coating concentration for the antigen was determined by performing a "checkerboard titration" with a reference positive and reference negative serum.

The antigen coated plates were washed 3 times in washing buffer using a Dynatech AM60 Multi-reagent washer, after which the plates were incubated for two hours with 200µl of blocking buffer. After washing, the relevant test sera diluted in incubation buffer were added to the plates and incubated for two hours in room temperature. Then the plates were washed again and a relevant enzyme- conjugated anti-species IgG immunoglobulin was added in the concentration 1/1000. The plates were incubated for two hours and washed. The substrate 2,2'-azino-bis(ethylbenzthiazoline-6-sulfonic acid) (ABTS) in substrate buffer, activated by hydrogen peroxide, was added and the reaction allowed to develop prior to reading in a spectrophotometer at 414nm.

### Potassium alum adjuvant preparation

10% aqueous solution of potassium alum (AIK(SO₄)₂ * 12 H₂O) was prepared by using filtered distilled water. 22.8ml of 250mM NaOH was added to 10ml of the potassium alum solution and stirred vigorously. The suspension was centrifuged at low speed (1500g) for ten minutes. After removal of the supernatant the pellet was washed twice in isotonic saline (centrifuged twice at 1500g for 5 minutes). Finally the pellet was resuspended in isotonic saline to 10ml and stored at 4°C.

### Immunization of Mice with CTF

The method used was that described by Darani et al, Parasitology, 115, (1997) 237-247.

### Preparation of Antisera

### A) Preparation of rabbit serum (BR67)

Three polyclonal rabbit sera were used to probe immunoblots of recombinant proteins. Serum BR67 was raised by immunising a rabbit with strips of nitrocellulose paper (NCP) carrying a 27 kDa S. mansoni larval protein which had been identified as the S. mansoni cercarial elastase. The antigen was purified onto NCP by electroblotting after protein separation by SDS-PAGE (as described below). The rabbit was immunised with excised strips of NCP carrying the protein dissolved in dimethyl sulphoxide and emulsified in Freund's complete adjuvant.

Serum 1093X was raised by immunising a rabbit subcutaneously with glutathione-S-transferase (GST) which had been purified from S. japonicum worm homogenate by affinity chromatography on glutathione-conjugated agarose and emulsified in Freund's complete adjuvant. When tested by Western blotting, the antiserum reacted with equal intensity against both the 26 kDa and 28 kDa S. japonicum GST isoenzymes.

As a negative control, serum BR114 was obtained from a rabbit immunised with horse albumin (Sigma) in Freund's adjuvant. All antisera were blocked by treating with total E. coli lysate (Sigma) for 15 hours at room temperature, centrifuging at 10,000g and discarding the resulting pellet.

### B) Rabbit immunised with S. haematobium 27kDa elastase

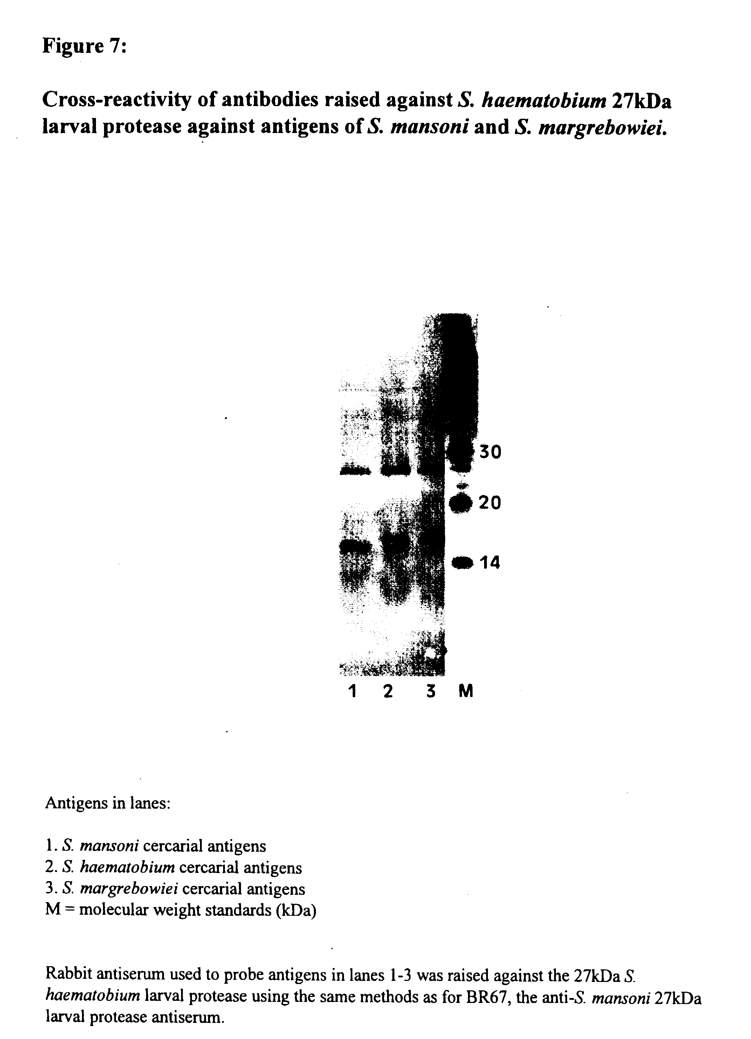

A serum was raised against the 27kDa elastase of S. haematobium using the same methods as for preparation of serum BR67 against S. mansoni 27kDa elastase as described in Materials and Methods. Figure 7 shows that the anti-S. haematobium elastase serum crossreacts with 27kDa molecules of both S. mansoni and S. margrebowiei.

### Example 1 - Cloning and structure of the Cercarial Elastase gene of S. mansoni and Exons Thereof.

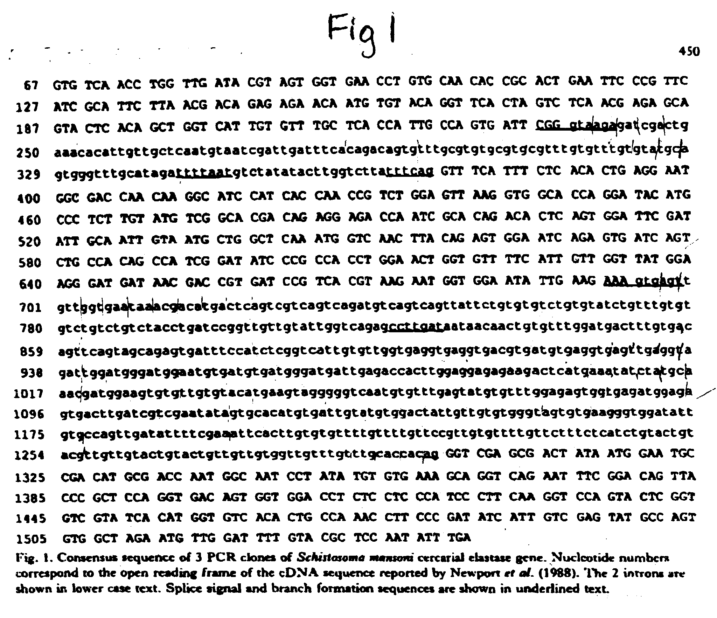

The consensus sequence of 3 PCR clones of the cercarial elastase gene of S. mansoni is shown in Figure 1 (Seq. I.D. 1). The nucleotide numbers correspond to the open reading frame of the cDNA sequence reported by Newport et al (J. Biol. Chem., 263, (1988), 13179-13184). The 2 introns are shown in lower case text. Splice signal and branch formation sequences are shown in underlined text.

Figure 1 identifies the three exons of the gene corresponding to amino acid residues 28-78 (exon 1), 79-184 (exon 2) and 185-265 (exon 3).

### Cloning the Cercarial Elastase Gene

Approximately 2µg of a 1477bp DNA fragment amplified from genomic DNA extracted from S. mansoni cercariae, was subjected to phenol/chloroform extraction and ethanol precipitation, as above. The amplified DNA was then treated with Klenow fragment of *E*. *coli* DNA polymerase I and nucleotides to give blunt-ended fragments. The total volume of reaction was 30µl, containing 30mM Tris.HCl [pH 7.2], 10mM MgSO₄, 1mM DTT, 0.2mM each of dATP, dCTP, dGTP and dTTP, 1µg DNA and 2 units of Klenow. The solution was incubated at 37°C for 5 minutes, then at 70°C for 10 minutes. The DNA was ligated into *Smal*-digested pT7T3α18 vector in a total volume of 20µl, containing 0.5µg of digested plasmid DNA, 0.2µg of digested insert DNA, 30mM Tris.HCl [pH 7.8], 10mM MgCl₂, 10mM DTT, 1.5mM ATP, 4% (w/v) PEG 8000 and 2 units of T4 Ligase. The solution was incubated at 16°C overnight, then at 70°C for 10 minutes.

Transformation was performed using the calcium chloride method into *E*. *coli* strain XL1-Blue. Selected transformants were screened for the cercarial elastase gene insert by the use of cells as a source of DNA for PCR. A small number of cells were resuspended in 100µl of sterile filtered water, placed in a water bath at 100°C for 3 minutes, and 10µl used in PCR as above, in place of genomic DNA.

Three independent clones, designated as pHP1, pHP2 and pHP3, were subjected to dideoxynucleotide sequencing using a Sequenase Version 2.0 kit (Amersham USB, Bucks., UK) and M13 universal sequencing primers (Genosys). Additional data was produced by sub-cloning *EcoRV*-digested fragments of the elastase insert into Smal-digested pT7T3α18, and performing DNA sequences as above. Resulting data was aligned with the previously described cercarial elastase cDNA clone sequence (Newport *et al.,* reference above), using Gene Jockey Sequence Processor (Biosoft, Cambridge, UK) on an Apple Macintosh computer.

### Cloning the Three Exons of the Gene

Oligonucleotide primers were designed to align with the 3' and 5' ends of the three exons of the gene, amino acid residues 28-78, 79-184 and 185-265 (numbers based on the open reading frame). Restriction sites were incorporated into the primers to facilitate cloning (first exon - primers 3 and 4, with *Bam*HI and *Xba*I; second exon - primers 5 and 6, with SacI and *Eco*RI; and third exon - primers 7 and 2, with *Xhol* and a Klenow-filled blunt end). PCR was used to amplify the exons from S. mansoni genomic DNA. The total reaction volume was 50µl, containing 10mm Tris.HCI [pH 9.0], 3mM MgCl₂, 50mM KCI, 0.1% (v/v) Triton X-100, 60 pmol of each primer, 0.05µg of genomic DNA, 0.25mM each of dATP, dCTP dGTP and dTTP and 0.5 units of *Taq* DNA Polymerase. Negative control tubes were set up as above. Amplification was performed on the thermocycler, with an initial denaturing step at 95°C for 5 minutes, then for 25 cycles (92°C for 30 seconds, 50°C for 1 minute, 74°C for 1 minute).

The amplified fragments were subjected to phenol/chloroform extraction and ethanol precipitation as above. The fragments were digested with appropriate restriction enzymes and ligated into double digested pGEX-KG plasmid vector, in a total volume of 20µl, containing 0.5µg of digested plasmid DNA, 0.2µg of digested insert DNA, 30mM Tris.HCl [pH 7.8], 10mM MgCl₂, 10mM DTT, 1.5mM ATP, 4% (w/v) PEG-8000 and 2 units of T4 Ligase. Transformation and screening of colonies by the use of PCR was performed as above.

Clones of the three exons were digested with BamHI and *Hind*III, and the inserts were sub-cloned into *Bam*HI-*Hind*III digested pT7T3α18 vector. DNA sequencing of the inserts were performed as before. The clones were designated as pGEXON1, pGEXON2 and pGEXON3 respectively.

### Example 2 Production of Fusion Proteins of Exons 1, 2 and 3

Aliquots of *E*. *coli* strain JM109 were transformed separately as above with each of the three constructs pGEXON1, pGEXON2 and pGEXON3. Single colonies of each were grown overnight at 37°C in 5ml of 2-YT broth containing 50µg/ml ampicillin and 50µg/ml methicillin. An aliquot of 1ml of each culture was added to 50ml of 5-YT broth containing antibiotics as above. After incubation at 37°C until an OD600 of 1.0 was reached, the bacteria were induced by adding filter sterilised IPTG to a final concentration of 0.5mM. Cells were grown for a further 2 hours at 37°C. Control cultures of cells transformed with pGEX-KG and untransformed cells, were also grown and induced as above. Samples of 200µl were taken from the induced bacterial cell cultures and centrifuged at 5,000g for 5 minutes. The cell pellets were resuspended in 50µl of cell lysis buffer (2% (w/v) SDS, 10mM EDTA [pH 8.0], 50µg/ml PMSF, freshly prepared) and boiled for 3 minutes. 15µl of each cell lysate was added to 15µl of loading buffer (100mM Tris.HCl [pH 6.8], 200mM DTT, 20% (v/v) glycerol, 10mM EDTA [pH 7.0], 4% (w/v) SDS, 0.2% (w/v) bromophenol blue). The samples were boiled for a further 3 minutes, then electrophoresed on a 10% (w/v) SDS polyacrylamide gel and stained with 0.5% (w/v) Coomassie blue.

### Example 3 Production of cDNA Fusion Protein

### A. DNA Constructs

A cDNA clone of the Schistosoma mansoni cercarial elastase gene in plasmid vector pBluescript II SK was obtained from Dr. J. Khalife from INSERM, Institut Pasteur, Lille, France. This clone is designated as pCEL.

Construct pGEXON1 is a clone of the first exon of the S. mansoni cercarial elastase gene in pGEX-KG vector as described in Example 1.

### B. Production of Construct pGEXEL

### (i) Summary of Plasmid Construction

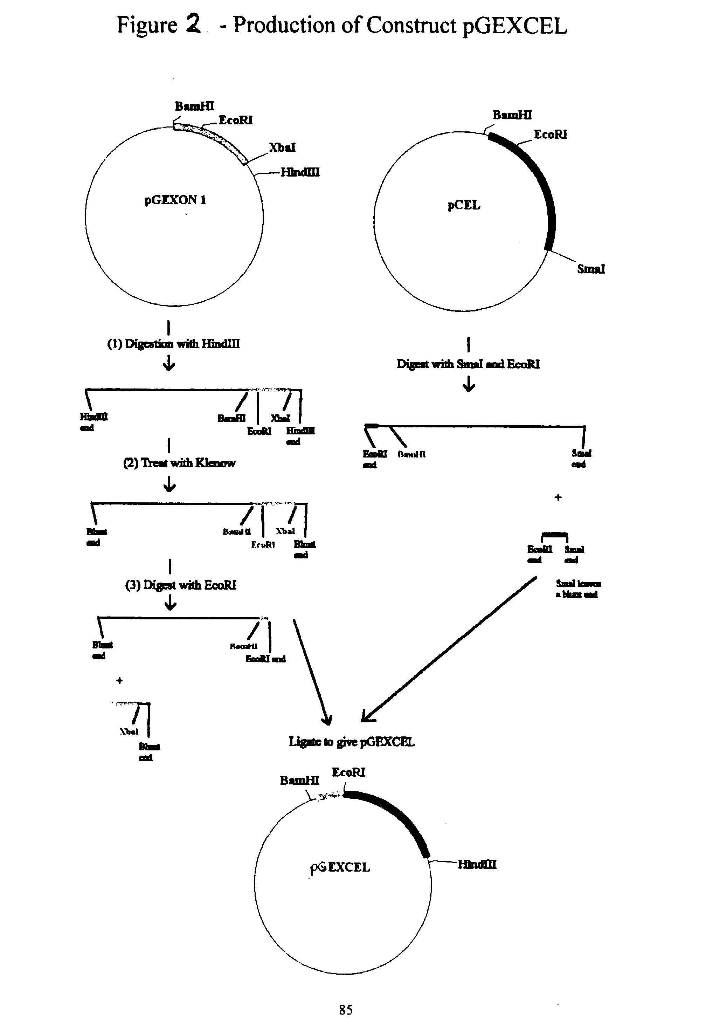

The method used to construct pGEXEL, pGEX-derived plasmid containing a cDNA clone of the S. mansoni elastase gene in the correct reading frame, is shown in Figure 2. The process was facilitated by the use of pGEXON1, a previously constructed clone of the first exon of the elastase gene inserted in the correct reading frame into a pGEX expression vector (described in Example 1).

The construct pGEXON1 was treated with restriction enzymes to remove the first exon insert except for 36 nucleotides at the 5' end of the insert which were left in the correct reading frame in the linear plasmid. Appropriate restriction enzymes were used to excise an S. mansoni cercarial elastase cDNA fragment, minus 36 nucleotides at the 5' end of the mature protein coding sequence, from a Bluescript plasmid vector. The cDNA fragment was then ligated into the treated pGEXON1 linear DNA to produce a pGEX clone of the full-length elastase gene in the correct reading frame.

### (ii) Method of Plasmid Construction

A sample of the construct pCEL (2µg) was digested with the restriction enzymes *SmaI* and *Eco*RI and the digested DNA fragments were separated on a 1% (w/v) agarose electrophoresis gel. The agarose gel was stained in methylene blue solution and a 675 bp DNA fragment was purified from the stained agarose. The resulting DNA pellet was resuspended in 20µl of TE buffer (10mM Tris.HCl [pH8.0], 1mM EDTA [pH 8.0].

A sample of construct pGEXON1 (2µg) was digested with *Hind*III. After electrophoresis of the digested DNA, a 5100bp fragment was purified from a methylene blue stained 1% (w/v) agarose gel, using the method described above. The purified DNA treated with Klenow fragment of *E*. *coli* DNA Polymerase I to give blunt-ended fragments. The total reaction volume was 30µl, containing leg of the purified DNA, 30mM Tris.HCl [pH 7.2], 10mM MgSO₄, 1mM DTT, 0.2mM each of dATP, dCTP and dTTP and 2 units of Klenow fragment. The solution was incubated at 37°C for 5 mins, then at 70°C for 10 mins. The treated DNA was then digested with EcoRI.

The 675bp fragment from pCEL was ligated into the treated pGEXON1 vector, in a total volume of 20µl, containing 0.5µg of vector DNA, 0.2µg of insert DNA, 30mM Tris.HCl [pH7.8], 10mM MgCl₂, 10mM DTT, 1.5mM ATP 4% (w/v) PEG 8000 and 2 units of T4 ligase. The solution was incubated at 16°C overnight, then at 70°C for 10 minutes. The ligation was transformed into *E*. *coli* strain XL1-Blue using the CaCl₂ competent cell method. Clones were screened for the presence of the elastase gene by PCR. A clone of the cercarial elastase cDNA fragment in pGEXON1 was designated as pGEXEL.

### C. Production of Fusion Proteins

Aliquots of two different *E*. *coli* strains were individually transformed with the constructs pGEXCEL and pGEX-KG. Single colonies of each were grown overnight at 37°C in 5ml of 2-YT broth containing 50µg/ml ampicillin, 50µg/ml methicillin and 30µg/ml kanamycin. An aliquot of 1ml of each culture was added to 50ml of 5-YT broth containing antibiotics as above. After incubation at 37°C until an OD600 of 1.0 was reached, the bacteria were induced by adding filter sterilised IPTG to a final concentration of 0.5mM. Cells were grown for a further 3 hours at 37°C.

Samples of 200µl were taken from the induced bacterial cell cultures at 30 minute intervals after the time of induction. The samples were centrifuged at 5,000g for 5 minutes and analysed using SDS-PAGE. An LKB 2202 Ultroscan laser densitometer and the computer program Apple Gelscan were used to measure the relative intensities of recombinant and native protein bands on the polyacrylamide gels. For cells containing the construct pGEXCEL, native bacterial proteins with molecular weights of approximately 74 kDa, 70 kDa, 62 kDa and 42 kDa were analysed and compared with the expressed recombinant protein. For cells containing the vector pGEX-KG, native bacterial proteins with molecular weights of approximately 52 kDa, 42 kDa, 35 kDa and 32 kDa were selected for analysis in addition to the recombinant protein.

### Example 4 - Purification of Fusion Proteins

The recombinant proteins obtained in accordance with Examples 2 and 3 above were purified and designated as Sj26 (unfused GST), Sm30-GST (cercarial elastase fused to GST), Sm30 Ex1-GST, Sm30 Ex2-GST and Sm30 Ex3-GST (peptides derived from the individual first, second and third exons respectively of the cercarial elastase gene fused to GST. The GST (glutathione-S-transferase) is introduced using a GST fusion vector, pGEX-KG described by Guan and Dixon J E (Analytical Biochemistry, 192: 262-267, 1991). This is a 5.0 kb plasmid, containing a β-lactamase gene to facilitate selection of transformants, and a multiple cloning site into which the foreign DNA fragment is inserted immediately downstream of the sequence encoding the 26 kDa GST from Schistosoma japonicum. The expression of the GST gene is tightly regulated by a *trp-lac* (tac) promoter, which can be induced by derepression with β-D-isopropyl-thiogalactopyranoside (IPTG). An over-expressed *lacI* gene is also present on the plasmid vector, to mediate repression of the *tac* promoter until IPTG induction, independent of the *lacI* status of the E.coli host. This is important in preventing the expression of proteins that may be toxic to the cells, until a desired cell density is reached and induction is instigated. A plasmid map of pGEX-KG is shown in Appendix C of the above referenced paper.

Expressed GST fusion proteins may be purified by affinity chromatography, utilising the ability of enzymatically active GST to bind reversibly with high affinity to immobilised reduced glutathione. Once the fusion protein has been purified, the protein of interest can be cleaved from the GST at a thrombin cleavage site just upstream of the fusion junction. However, in this study, the fusion proteins were used in an uncleaved form, as the GST portion is very immunogenic, causing a strong antibody response. (For reference see Oettinger HF, Biotechniques, 12, (1992), 544-549.) This has been shown to be particularly advantageous when the protein of interest is a poor immunogen.

The recombinant fusion proteins were purified by the following method which describes the purification of Sm30 Ex1-GST.

Cells from a 100ml culture containing the recombinant protein SM30 Ex1-GST were lysed in 10ml of cell lysis buffer (as described above). The insoluble fraction was resuspended in 30ml of PBS solution (137mM NaCl, 2.68mM KCl, 4.3mM NaH₂PO₄, 1.47mM KH₂PO₄, pH7.4), centrifuged at 15000g for 30 minutes at 4°C, and the step repeated once with PBS solution and twice with wash buffer (50mM glycine [pH9.0], 50mM NaCl, 3M urea, 2mM DTT, 1mM PMSF). The insoluble pellet was resuspended in 3ml of column start buffer (50mM glycine [pH 9.0], 50mM NaCl, 8M urea, 2mM DTT, 1mM PMSF) and stirred at room temperature for 30 minutes. The mixture was centrifuged at 12000g for 15 minutes at room temperature. The supernatant was passed through a 0.22µm disposable filter (Schleicher and Schuell, New Hampshire, USA) and then ion-exchange chromatography was performed.

A 2ml syringe, plugged with siliconised glass wool, was packed with 1.5ml of High Performance Q Sepharose (Pharmacia). The column was washed by passing through 50ml of 15%(v/v) isopropanol, using a P-1 peristaltic pump (Pharmacia) with a flow rate of 60ml/hour. A 10ml volume of column start buffer (as above), 2ml of activation buffer (50mM glycine [pH 9.0], 1M NaCl, 8M urea, 2mM DTT, 1mM PMSF) and a further 15ml of start buffer were sequentially passed through the column, maintaining a flow rate of 40ml/hour. The filtered protein solution was then passed through, followed by 30ml of column start buffer.

The bound proteins were eluted from the column using a linear ionic strength gradient, from 50mM NaCl to a final concentration of 500mM over 20 column volumes of start buffer. A series of lml samples were collected from the column and a 20µl aliquot of each was tested for the presence of protein using the Bradford assay. Samples containing protein were also analysed by SDS-PAGE to locate the expressed GST-fusion protein.

Samples containing the recombinant protein and minimal host contaminants were pooled and dialysed. Visking dialysis tubing with a 19.0mm diameter (Medicell International Limited, London, UK) was pre-treated by boiling in 2% (w/v) sodium bicarbonate, 1mM EDTA, for 10 minutes and then autoclaving in distilled water. Dialysis was then performed for 4 days at 4°C, with 8-hourly changes of dialysis buffer (50mM glycine [pH 9.0], 25mM NaCl, 2mM DTT, 1mM PMSF). Following dialysis, the protein solution was centrifuged at 12000g for 10 minutes and aliquots of the resulting supernatant were analysed by SDS-PAGE.

The ion-exchange Sepharose was re-generated by washing with 2 volumes of 2M NaCl, 5 volumes of 30% isopropanol and then 10 volumes of the appropriate column start buffer. The resin was stored in 20% ethanol at 4°C until required.

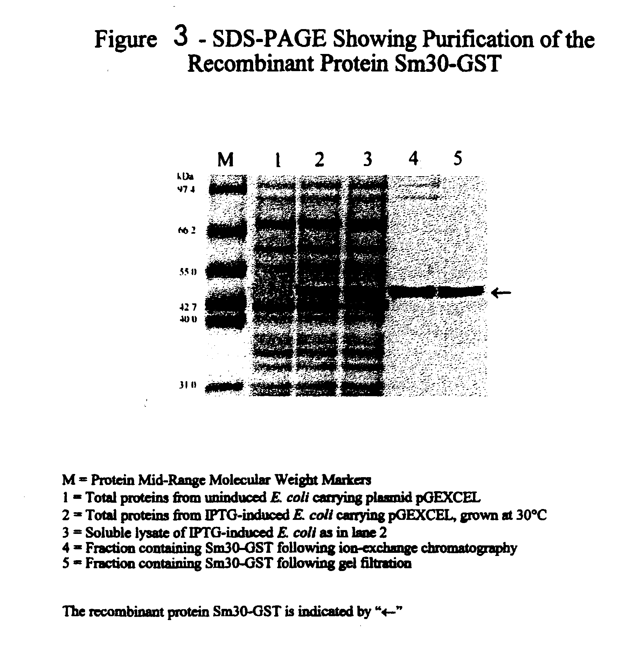

The SDS-PAGE results for the purified recombinant protein Sm30-GST are shown in Figure 3. These were obtained by SDS-PAGE Method A.

### Example 5 - Immunisation of a Rabbit with a Recombinant Fusion Protein Sm30-GST

### a) Antigen Preparation

An IPTG-induced bacterial lysate containing Sm30-GST was subjected to protein separation by SDS-PAGE and transferred onto nitro-cellulose paper (NCP) by electroblotting, using methods above. The lateral edges of the immunoblot were removed, incubated in Protogold protein stain overnight and then used to locate the recombinant elastase GST-fusion protein in the remaining immunoblot. An NCP strip containing the recombinant protein was excised and dissolved in dimethyl sulphoxide (DMSO) to a final protein concentration of 0.2mg/ml. Emulsions were prepared by mixing the antigen in a 1:1 ratio with either Freund's Complete Adjuvant (FCA) or Freund's Incomplete Adjuvant (FIA).

### b) Active Immunisation

A sandy half-lop rabbit was actively immunised with the prepared antigen. The animal received an initial lml injection, containing 100µg of antigen in FCA, administered subcutaneously at multiple sites on the flanks. Following a 21 day period, 8 booster injections of 50µg of antigen in FIA were administered at fortnightly intervals.

### c) Collection of Blood Samples

During the course of the immunisation experiment, blood samples of up to 10ml were taken at monthly intervals from the lateral ear vein of the rabbit. The blood was allowed to clot for 1 hour and then incubated at 4°C overnight. The sample was centrifuged at 5000g for 5 minutes, and the pellet was discarded. The remaining serum was stored at-20°C. Antibody responses to a range of antigens were tested by Western blotting, using methods described above.

After 8 booster injections had been administered, the rabbit was serially bled until approximately 100ml of serum had been collected. The animal was then exsanguinated under ether anaesthesia.

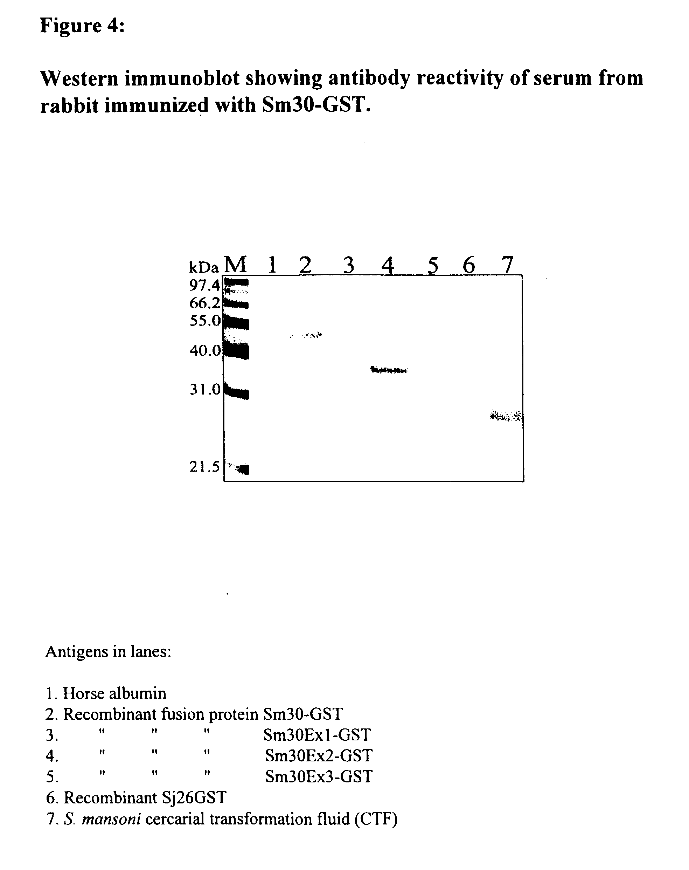

The results are shown in Figure 4.

These results show that serum from a rabbit immunised with Sm30-GST was reactive against the native form of the 27/28 kDa protease, the expression product containing recombinant second exon (Sm30 Ex2-GST) and the immunising construct. However, this serum did not react against either Sm30 EX1-GST or Sm30 EX3-GST, ie. the fusion proteins containing the two outer exons of the protease

### Example 6 - Use of Fusion Protein to Produce Immune Reaction in Mice

Mice were immunised with CTF and recombinant fusion proteins as described above.

Using the method of Western blotting described above S. mansoni cercarial antigens were probed with sera of individual mice injected with
A. CTF containing native cercarial protease and
B. recombinant cercarial protease Sm30-GST

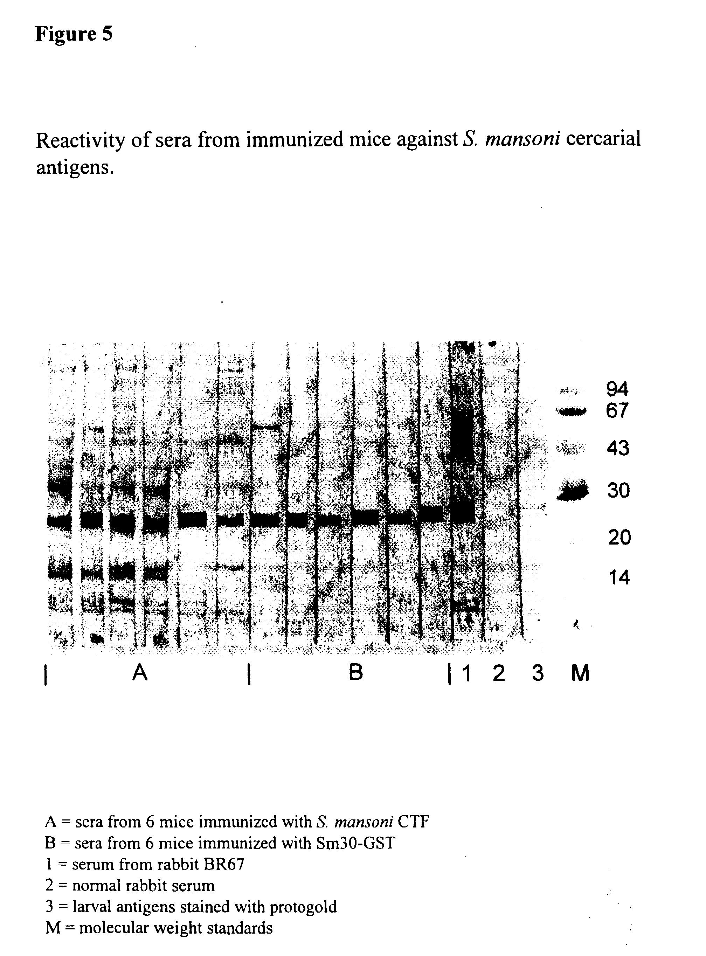

The results are shown in Figure 5 where
Lane 1 -probed with BR67
Lane 2 -probed with normal rabbit serum
Lane 3 -stained with Protogold
(MW = molecular weight marker)

Figure 5, Group A shows the results for 6 mice from a larger group that had been repeatedly immunized with native cercarial protease that is present in cercarial transformation fluid (CTF). The preparation of CTF is given by Darani et al, Parasitology (1997), 115, 237-247, which also shows that only a minority of mice immunized with CTF produced antibody to the elastase and that only those mice which produce antibody to the elastase are protected against schistosome infection.

In contrast, Figure 5, Group B shows sera from a group of mice immunized with Sm30-GST, all of which responded to the native elastase.

Thus the results show that, in contrast to immunisation with native protein, when only a minority of mice produce sufficient antibody, all mice in the group of 6 immunised with the "full length" fusion protein Sm30-GST produced antibody which reacted against the native protein. The antigen against which the sera were tested was S. mansoni cercarial homogenate prepared as described in Example 8 below.

### Example 7 - Immunisation of Mice via intraperitoneal route

Table 1 below gives the results of (1) worm burden and (2) tissue egg counts of mice injected with (A) recombinant cercarial protease, (B) CTF and (C) alum.
(1)

| Mouse group and number | Mean | SD | Worm burden reduction | P |
|---|---|---|---|---|
| Group A (13) | 41.1 | 16.4 | 43.6% | P<0.01 |
| Group B (12) | 57.3 | 24.6 | 21.2% | ns |
| Group C (6) | 72.8 | 12.8 | - | - |

(2)

| Mouse group | Mean | SD | Tissue egg reduction | P |
|---|---|---|---|---|
| Group A (13) | 20954 | 8293 | 47.5% | P<0.01 |
| Group B (12) | 27720 | 10625 | 30.5% | - |
| Group C (6) | 39933 | 12711 | - | - |

ns = not significant

The worm burden results were obtained as described above.

The number perfusable adult worms and of eggs in tissues of the infected mice were estimated as described in Materials and Methods.

It will be seen from the Tables above that mice immunized with SM30-GST were significantly protected against a S.mansoni challenge infection.

### Example 8 - Cross reactivity of Sm30-GST

Experiments were carried out to demonstrate the cross-reactivity of Sm30-GST with S. haematobium elastase and S. margrebowiei elastase in mice and rabbits.

A) mice immunised with Sm30-GST - reactivity with S. haematobium elastase.

The Western blotting technique described in Method B was employed to test how S. mansoni and S. haematobium cercarial antigens reacted with sera of individual mice injected with:
A - CTF containing native cercarial protease
B - recombinant cercarial protease Sm30-GST

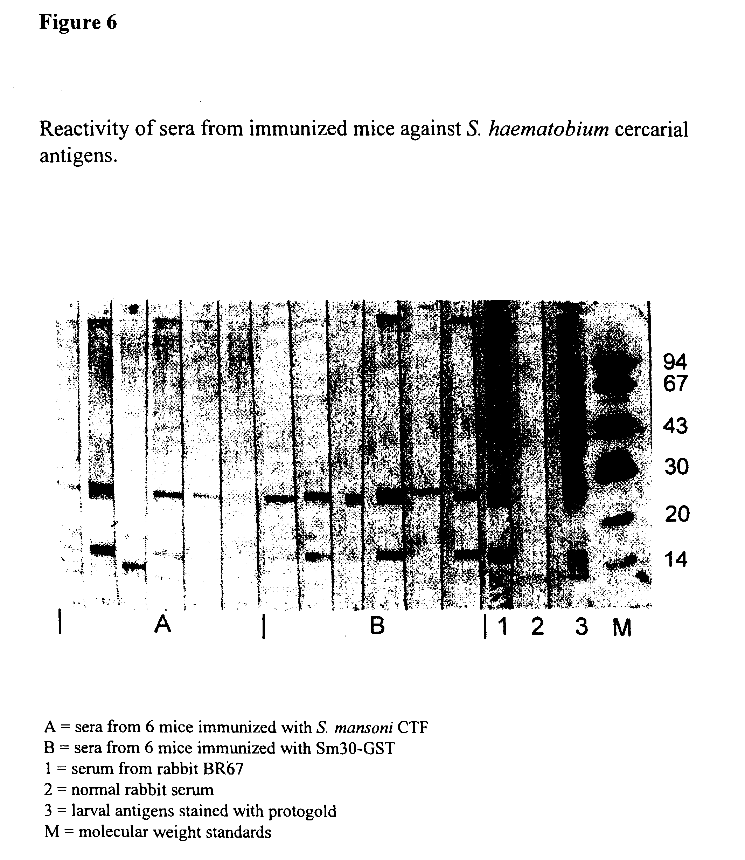

The results are shown in Figure 6 where:
Lane 1 - probed with BR67 (prepared as described in Materials and Methods)
Lane 2 - probed with normal rabbit serum
Lane 3 - Stained with Protogold
(MW = molecular weight markers)

The results in Figure 6 show that antisera from mice immunized with Sm30-GST react against a 27 kDa antigen in extracts of S. haematobium cercariae.

The reactivity of the antisera raised against the 27 kDa S. haematobium larval antigen was tested using extracts of whole cercarial homogenates of S. mansoni, S. haematobium and S. margrebowiei prepared as described in Materials and Methods.

Figure 7 shows that the anti-S.haematobium elastase serum crossreacts with 27 kDa molecules of both S. mansoni and S. margrebowiei.

### Example 9 - Immunization of Mice with Exon II

The following antigen preparations were used: Alum and PBS (group A), cercarial transformation fluid (group B), Sj26 (group C), Sm30-GST (group D), Sm30 EX1-GST (group E), Sm30 EX2-GST (group F) and Sm30 Ex3-GST (group G).

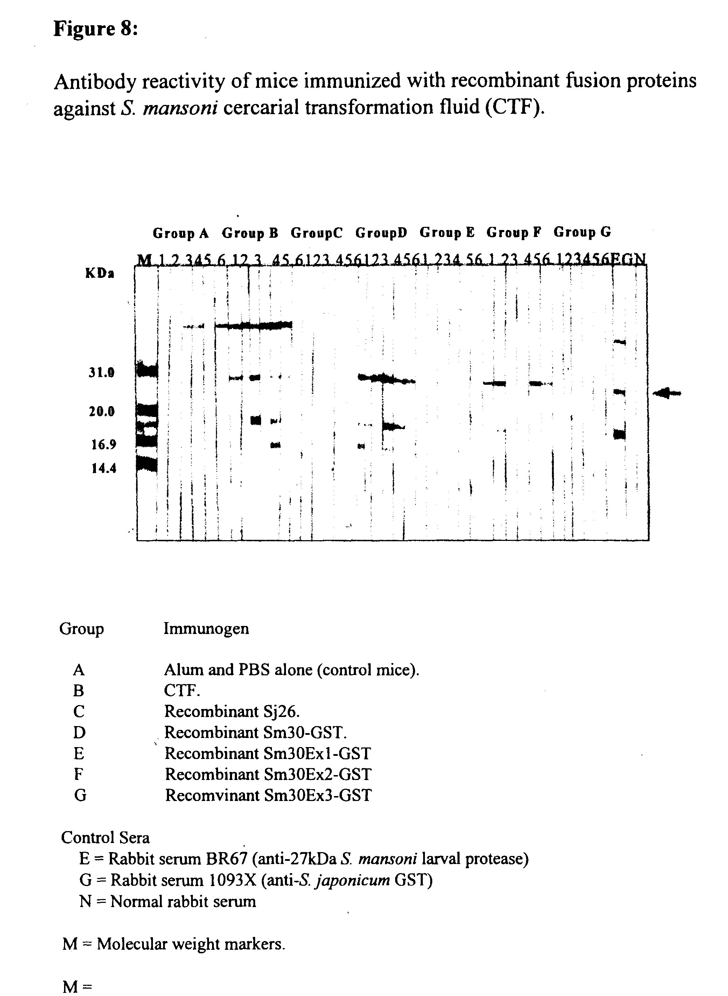

The serological results of immunizing groups of mice with recombinant protein containing the full length elastase (Sm30-GST) and with fusion proteins of the 3 respective exons individually are shown in Figure 8.

The Western blot confirms that exon 2 is more immunogenic than exons 1 and 3 in terms of inducing antibodies which react with the native form of the molecule.

### Annex to the description

Sequence Listings

Information for SEQ ID1

i) Sequence characteristics

A Length: 1478

B Type: Nucleic acid

C Strandedness: Single

ii) Sequence description

67 GTG TCA ACC TGG TTG ATA CGT AGT GGT GAA CCT GTG CAA CAC
CGC ACT GAA TTC CCG TTC

127 ATC GCA TTC TTA ACG ACA GAG AGA ACA ATG TGT ACA GST TCA
CTA GTC TCA ACG AGA GCA

187 GTA CTC ACA GCT GGT CAT TGT GTT TGC TCA CCA TTG CCA GTG
ATT CGG gtaagagatcgactg

250
aaacacattgttgctcaatgtaatcgattgatttcacagacagtgtttgcgtgtg
cgtgcgtttgtgtttgtgtatgca

329 gtgggtttgcatagatttaatgtctatatacttggtcttatttcag GTT TCA
TTT CTC ACA CTG AGG AAT

400 GGC GAC CAA CAA GGC ATC CAT CAC CAA CCG TCT GGA GTT AAG
GTG GCA CCA GGA TAC ATG

460 CCC TCT TGT ATG TCG GCA CGA CAG AGG AGA CCA ATC GCA CAG
ACA CTC AGT GGA TTC GAT

520 ATT GCA ATT GTA ATG CTG GCT CAA ATG GTC AAC TTA CAG AGT
GGA ATC AGA GTG ATC AGT

580 CTG CCA CAG CCA TCG GAT ATC CCG CCA CCT GGA ACT GGT GTT
TTC ATT GTT GGT TAT GGA

640 AGG GAT GAT AAC GAC CGT GAT CCG TCA CGT AAG AAT GGT GGA
ATA TTG AAG AAA gtgagtt

701
gttggtgaataaacgacatgactcagtcgtcagtcagatgtcagtcagttattct
gtgtgtctgtgtatctgtttgtgt

780
gtctgtctgtctacctgatccggttgttgtattggtcagagccttgataataaca
actgtgtttggatgactttgtgac

859
agttcagtagcagagtgatttccatctcggtcattgtgttggtgaggtgaggtga
cgtgatgtgaggtgagttgaggta

938
gattggatgggatggaatgtgatgtgatgggatgattgagaccacttggaggaga
gaagactcatgaaatatctatgca

1017
    aacgatggaagtgtgttgtgtacatgaagtagggggtcaatgtgtttgagtatgt
gtttggagagtggtgagatggaga

1096
    gtgacttgatcgtcgaatatagtgcacatgtgattgtatgtggactattgttgtg
tgggtagtgtaaagggtggatatt

1175
    gtgccagttgatattttcgaaattcacttgtgtgttttgttttgttccgttgtgt
tttgttctttctcatctgtactgt

1254acgttgttgtactgtactgttgttgtggttgtttgtttgcaccacag GGT CGA
GCG ACT ATA ATG GAA TGC

1325CGA CAT GCG ACC AAT GGC AAT CCT ATA TGT GTG AAA GCA GGT
CAG AAT TTC GGA CAG TTA

1385CCC GCT CCA GGT GAC AGT GGT GGA CCT CTC CTC CCA TCC CTT
CAA GGT CCA GTA CTC GGT

1445GTC GTA TCA CAT GGT GTC ACA CTG CCA AAC CTT CCC GAT ATC
ATT GTC GAG TAT GCC AGT

1505GTG GCT AGA ATG TTG GAT TTT GTA CGC TCC AAT ATT TGA

Information for SEQ ID2 (Exon 2)

i) Sequence characteristics

A. Length: 106

B. Type: Protein

C. Strandedness: Single

D. Topology: Linear

ii) Sequence description

1 VSFLTLRNGD QQGIHHQPSG VKVAPGYMPS

CMSARQRRPI AQTLSGFDIA IVMLAQMVNL

61 QSGIRVISLP QPSDIPPPGT GVFIVGYGRD

DNDRDPSRKN GGILKK

Information for SEQ ID3
i) Sequence characteristics
A. Length: 16
B. Type: Protein
C. Strandedness: Single
D. Topology: Linear

ii) Sequence description
V G Y G R D D N D R D P S R K N

## Claims

1. A vaccine comprising a recombinant fusion protein capable of eliciting immunity against Schistosoma parasites, comprising the 27/28 kDa cercarial elastase sequence of S. mansoni or an active fragment, homologue or variant thereof, fused to a suitable bacterial, phage or viral protein, together with a pharmaceutically acceptable excipient or carrier.

2. A vaccine according to claim 1 wherein the fusion protein comprises the amino acids coding for the nucleotide of the cercarial elastase gene of S. mansoni (Seq. I.D. 1) or a homologue or variant thereof.

3. A vaccine according to claim 1 wherein the fusion protein comprises at least the amino acid sequence coding for exon 2 of the S. mansoni cercarial elastase gene as herein defined (Seq. I.D. 2).

4. A vaccine according to any one of claims 1 to 3 wherein the fusion protein comprises at least amino acid residues 136 to 151 of the S. mansoni cercarial elastase molecule.

5. A vaccine according to any one of claims 1 to 4 wherein the fusion protein comprises a sequence of at least 16 amino acids including the sequence: V G Y G R D D N D R D P S R K N (Seq. I.D. 3) fused to a suitable bacterial, phage or viral protein.

6. A vaccine according to any one of the preceding claims wherein the fused protein is a glutathione-S-transferase.

7. A vaccine according to claim 6 wherein the fused protein is the 28 kDa glutathione-S-transferase of S. japonicum.

8. A vaccine according to any one of the preceding claims adapted for oral administration or administration by injection.

9. A vaccine according to any one of the preceding claims capable of eliciting immunity in a human inoculated with the vaccine.

10. A vaccine according to claim 9 capable of eliciting immunity against S. mansoni and/or S. haematobium.
